# EUROPÄISCHE PATENTANMELDUNG

(11) **EP 4 389 040 A1**
(43) Veröffentlichungstag der Anmeldung: **26.06.2024**
(21) Anmeldenummer: 23216762.7
(22) Anmeldetag: 14.12.2023
(51) Int. Cl.: A61B 18/12

(54) **MODULARES ELEKTROCHIRURGIE-GENERATORSYSTEM**

(30) Priorität: 23.12.2022 US 202263434982 P
(71) Anmelder: Olympus Winter & Ibe GmbH, 22045 Hamburg (DE)
(72) Erfinder: Dietrich, Stefan, 14469 Potsdam (DE); Kwik, Anne, 14165 Berlin (DE); Ramin, Daniel, 14558 Nuthetal (DE); Fähsing, Thomas, 12305 Berlin (DE); Prezewowsky, Thomas, 14513 Teltow (DE); Schiddel, Stefan, 14532 Stahnsdorf (DE); Krüger, Jens, 15738 Zeuthen (DE); Arnold, Mario, 12157 Berlin (DE); Arras-Krempien, Rocco, 15712 Königs Wusterhausen/Zernsdorf (DE); Kühne, Wolfgang, 16552 Schildow (DE); Dijkstra, Jelle, 12205 Berlin (DE); Janich, Fabian, 14469 Potsdam (DE); Stopp, Fabian, 10318 Berlin (DE); Malchow, Imke, 24537 Neumünster (DE); Handrick, Veronika, 12059 Berlin (DE)
(74) Vertreter: Glawe, Delfs, Moll

(57) **Zusammenfassung**

Modulares Generatorsystem umfassend wenigstens zwei Generatortypen von Elektrochirurgie-Generatoren (1), die aus mehreren Generatorkomponenten zusammengesetzt sind und sich bezüglich ihrer Ausrüstung und/oder Funktionen unterscheiden. Die Generatorkomponenten umfassen eine Leistungsversorgung (31) und einen davon gespeisten Inverter (34) zur Erzeugung einer Ausgangsenergie, die zu mindestens einem Ausganganschluss (19) zum Anschluss eines elektrochirurgischen Instruments (99) geführt ist. Erfindungsgemäß sind die Generatortypen jeweils in Modulbauweise ausgeführt mit einer Mehrzahl von Modulen: (i) mindestens ein einheitliches Basismodul (2) mit einer Kommunikationseinheit (40) zur Intermodulkommmunikation, und (ii) mindestens ein Individualmodul (3), das je nach Generatortyp unterschiedlich ist. Individualmodule (3) sind ausgeführt bspw. als Modul für die Leistungsversorgung (31), Modul für den Inverter (34), ein Modul (35) für den Ausgangsanschluss (19). Verschiedene Generatortypen unterscheiden sich hinsichtlich ihrer Individualmodul-Ausstattung. Dank der Zweiteilung des Modulkonzepts in einheitliches Basismodul und sich unterscheidende Individualmodule kann mit einem Minimum der Anzahl verschiedener Module eine maximale Anzahl von Varianten

## Beschreibung

Die Erfindung betrifft ein modulares Generatorsystem umfassend wenigstens zwei Generatortypen von Elektrochirurgie-Generatoren. Die Generatortypen unterscheiden sich bezüglich ihrer Ausrüstung und Funktionen und sind jeweils zur Speisung mindestens eines elektrochirurgischen Instruments ausgebildet.

Bei der Elektrochirurgie oder Hochfrequenz-Chirurgie wird Energie in Form von hochfrequentem Wechselstrom mittels eines elektrochirurgischen Instruments, wie einem Elektroskalpell, in ein Gewebe des menschlichen Körpers eingetragen. Genutzt werden hierbei insbesondere Frequenzen von 200 kHz oder höher bis zu 4000 kHz, typischerweise um die 400 kHz. Mit der damit verursachten Erwärmung wird insbesondere das Gewebe geschnitten oder durchtrennt. Ein Vorteil hierbei liegt darin, dass gleichzeitig mit dem Schnitt auch eine Blutungsstillung durch Verschluss der betroffenen Gefäße erfolgen kann, und elektrochirurgische Instrumente für weitere Anwendungsarten in Betracht kommen, wie beispielsweise zur Koagulation.

Zur bedarfsgerechten Versorgung unterschiedlichster Indikationen bei Patienten werden Elektrochirurgie-Generatoren benötigt, die je nach dem vorgesehenen Anwendungsfeld ausgerüstet sind und entsprechend unterschiedliche Funktionen aufweisen. So gibt es Baureihen von unterschiedlichen Elektrochirurgie-Generatoren für Anwendungen im Bereich im allgemeinen Bereich sowie für Viszeralchirurgie, in spezielleren Bereichen wie für Hals/Nasen/Ohren, Urologie, Gynäkologie, Gastroenterologie, Pneumologie oder auch Phlebologie. Innerhalb dieser Baureihen gibt es wiederum Abstufungen für kleinere und größere Generatoren, leistungsfähigere und weniger leistungsfähige sowie Generatoren mit mehr oder weniger Funktionen. Während dies für den Patienten und für den Operateur von Vorteil ist in Bezug auf einen für die jeweilige Indikation passgenau zugeschnittenen Elektrochirurgie-Generator in der jeweils gewünschten Größe und mit dem jeweils gewünschten Funktionsumfang, so bedeutet dies für den Hersteller eine enorm hohe Anzahl unterschiedlicher Varianten.

Eine derartige Variantenvielfalt von Elektrochirurgie-Generatoren bereitzustellen ist ausgesprochen aufwendig. Dieser große Aufwand steht auch einer Modernisierung oder Erneuerung der Generatoren entgegen, da dies für die unterschiedlichen Baureihen und Größen in zwar ähnlicher, aber doch abweichender Form jeweils gesondert durchgeführt werden müsste. So vorteilhaft die Variantenvielfalt einerseits ist für die Nutzung eines umfassenden Generatorsystems, so nachteilig kann dies aber in Bezug auf fortschreitendende Technik sein, weil durch die Variantenvielfalt Anpassungen und Modernisierungen erschwert sind.

Der Erfindung liegt die Aufgabe zugrunde, ein verbessertes Generatorsystem zu schaffen, welches diese Nachteile vermeidet.

Die erfindungsgemäße Lösung liegt in den Merkmalen der unabhängigen Ansprüche. Vorteilhafte Weiterbildungen sind Gegenstand der abhängigen Ansprüche.

Bei einem Generatorsystem umfassend wenigstens zwei Generatortypen von Elektrochirurgie-Generatoren, die sich bezüglich ihrer Ausrüstung und/oder Funktionen unterscheiden, wobei die Generatortypen jeweils zur Speisung mindestens eines elektrochirurgischen Instruments ausgebildet sind und zusammengesetzt sind aus mehreren Generatorkomponenten umfassend eine Leistungsversorgung und einen Inverter, gespeist von der Leistungsversorgung zur Erzeugung einer Ausgangsenergie, die zu mindestens einem Ausganganschluss zum Anschluss des elektrochirurgischen Instruments geführt ist, ist erfindungsgemäß vorgesehen, dass die Generatortypen jeweils in Modulbauweise ausgeführt sind mit einer Mehrzahl von Modulen, die umfassen (i) mindestens ein bei den verschiedenen Generatortypen einheitliches Basismodul, das mindestens eine Kommunikationseinheit mit anderen Modulen umfasst, und (ii) mindestens ein Individualmodul, das je nach Generatortyp unterschiedlich ist, wobei das oder die Individualmodule ausgeführt sind zumindest als Modul für die Leistungsversorgung und/oder Modul für den Inverter und/oder mindestens ein Modul für den Ausgangsanschluss, wobei das mindestens eine Basismodul gleich ausgebildet ist für die verschiedenen Generatortypen, und die verschiedenen Generatortypen sich hinsichtlich ihrer Individualmodul-Ausstattung unterscheiden.

Die Erfindung basiert auf dem Gedanken, die Generatortypen jeweils in Modulbauweise auszuführen und so ein modulares Konzept zu schaffen, wobei dieses modulare Konzept verschiedene Module, aber auch verschiedene Arten von Modulen umfasst. So besteht als eine Modulart das Basismodul, welches einheitlich bei den verschiedenen Generatortypen eingesetzt ist. Hierbei bedeutet "einheitlich", dass sie im Wesentlichen gleich ausgebildet sind, insbesondere hinsichtlich des Hardware-Aufbaus, wobei unvermeidliche oder geringfügige Anpassungen vorgesehen sein können; es ist auch nicht ausgeschlossen, dass ID- oder Codiereinrichtungen bezüglich der Identität (z.B. Seriennummer) unterschiedlich sind. - Als andere Modulart bestehen Individualmodule, genauer gesagt mindestens ein Individualmodul, wobei der oder die Individualmodule sich zwischen den verschiedenen Generatortypen unterscheiden können. Dank der Zweiteilung des Modulkonzepts in einheitliches Basismodul und sich unterscheidende Individualmodule kann, wie die Erfindung erkannt hat, mit einem Minimum bezüglich der Anzahl verschiedener Module eine maximale Anzahl von Varianten erreicht werden zur Herstellung möglichst vieler unterschiedlicher Generatortypen.

Das Konzept des Basismoduls sorgt hierbei für eine gemeinsame und im Grunde unveränderliche Grundlage, auf welche sich die Individualmodule stets in gleicher Weise beziehen können. Dies betrifft insbesondere die erforderliche Kommunikation der Module, für die das Basismodul stets eine Kommunikationseinheit bereitstellt. Somit ist ganz unabhängig von der Art und Funktion bzw. Größe und Leistungsvermögen des Individualmoduls sichergestellt, dass dieses mit einer bekannten einheitlichen Grundlage in Gestalt des Basismoduls zusammenwirkt und an der Kommunikation mit dem Basismodul teilnehmen kann sowie, sofern vorhanden, auch mit anderen Individualmodulen. Es spielt hierbei keine Rolle, wie das jeweilige Individualmodul beschaffen ist oder welche Funktion es ausübt. Es kommt auch nicht darauf an, ob das Individualmodul elektrische Energie benötigt, wie im Fall eines Inverters zur Erzeugung der Ausgangsenergie, oder diese bereitstellt, wie im Fall einer Leistungsversorgung als Individualmodul. Auf diese Weise können nahezu universell durch Bestückung mit unterschiedlichen Individualmodulen unterschiedliche Generatortypen hergestellt werden. Somit können mit wenig Aufwand eine hohe Anzahl von Varianten erzeugt und angeboten werden.

Zunächst seien einige verwendete Begriffe erläutert:
Ein Elektrochirurgie-Generator ist zur Speisung von mindestens einem elektrochirurgischen Instrument mit Energie ausgebildet. In der Regel erfolgt dies mittels hochfrequenter Wechselspannung. Typische Frequenzbereiche liegen im Bereich zwischen 200 kHz und 4000 kHz. Die hochfrequente Wechselspannung kann ggf. Amplituden im Bereich der Hochspannung aufweisen, typischerweise bis zu 10 kV, vorzugsweise bis zu 4000 V.

Die von dem Elektrochirurgie-Generator bereitgestellte Leistung liegt typischerweise im Bereich zwischen 1 und 500 Watt, wobei die Lastimpedanz stark variieren kann und entsprechend sich Ausgangsspannung und Energieabgabe ebenso stark und rasch ändern können. Somit treten hohe dynamische Leistungsvariationen auf, aber es bestehen auch statische Leistungsvariationen zwischen verschiedenen Generatortypen, typischerweise für Leistungsanforderungen beginnen mit einigen Watt bis hin zu mehreren 100 W.

Unter einem Inverter, der zur Erzeugung der Ausgangsenergie herangezogen ist, wird im weiteren Sinn ein an sich beliebiger Hochspannungsgenerator verstanden. Es kann sich hierbei um einen freischwingenden Generator handeln, insbesondere in der Ausführung als Eintaktwandler, oder auch um einen gesteuerten Inverter im engeren Sinn, mit dem (hochfrequente) Ausgangsenergie mit einer vorgegebenen Frequenz und (Hochspannungs-)Amplitude erzeugt werden kann. Die erzeugte Energie wird typischerweise über eine Ausgangsenergieleitung zum Anschluss für das elektrochirurgische Instrument geführt.

Als unterschiedliche Generatortypen werden solche Elektrochirurgie-Generatoren angesehen, die sich hinsichtlich ihrer Ausrüstung und/oder Funktion unterscheiden. Das kann insbesondere bedeuten, dass sich die Generatortypen unterscheiden hinsichtlich der Art und der Anzahl der Anschlüsse für elektrochirurgische Instrumente (diese Anschlüsse werden fachsprachlich auch als "Sockets" bezeichnet), und/oder sie können sich unterscheiden in Bezug auf die zur Verfügung stehenden Betriebsarten bzw. Schneidarten (die fachsprachlich auch als "Modes" bezeichnet werden). Ferner können sich die Generatoren unterscheiden bspw. in Bezug auf ihren Inverter zur Erzeugung der Ausgangsenergie; diese können sich insbesondere unterscheiden durch die von ihnen abgegebene Leistung wie auch die Art der (Hoch-)Spannungserzeugung. Das bedeutet auch, dass die Generatortypen unterschiedlich groß sein können sowohl physisch wie auch in Bezug auf ihre Leistung.

Typischerweise bilden die Module jeweils eine physische Einheit für sich. Darunter wird verstanden, dass sie einzeln handhabbare (ein- oder mehrteilige) Körper bilden, der somit leicht zu handhaben und zu montieren ist. Ein eigenes Gehäuse hingegen weisen die Module typischerweise nicht auf, da sie es nicht benötigen; denn die Module sind in das eigentliche Gehäuse des Elektrochirurgie-Generators einzusetzen und von diesem geschützt. Davon zu unterscheiden sind auf oder an den Modulen vorgesehene abzuschirmende Bereiche, die mit einer entsprechenden Abschirmung ausgeführt sein können, die auch kastenartig ausgestaltet sein kann, wie es typischerweise bei Hochfrequenz-Komponenten der Fall ist. So kann mit Vorteil vorgesehen sein, dass auch das Gehäuse modular ausgeführt ist und in verschiedenen Größen (wie klein, mittel und groß) bereitgestellt ist, und es somit ähnlich wie ein (spezielles) Individualmodul behandelt werden kann.

Unter dem Oberbegriff "Module" werden sowohl Individualmodule wie auch das Basismodul (bzw. wenn mehrere Basismodule vorhanden sind, dann auch diese) verstanden.

Mit Vorteil ist das Basismodul - bzw. wenn mehrere vorgesehen sind, mindestens eines der Basismodule - als ein Verteilungsmodul ausgeführt, welches außer der Kommunikationseinheit eine Stromversorgungs-Verteilungseinheit aufweist. Es ist dazu ausgebildet, die für den Betrieb der Module erforderliche Leistung an die jeweiligen angeschlossenen Module zu verteilen und entsprechend mit den anderen Modulen über die Kommunikationseinheit zu kommunizieren. Das Basismodul braucht aber nicht zwingend als Verteilungsmodul ausgeführt sind, sondern zusätzlich oder alternativ können auch andere und/oder zusätzliche Basismodule vorgesehen sein. Hierbei kann es sich beispielsweise handeln um ein Displayansteuerungsmodul, ein internes Verteilungsmodul, und/oder ein Extern-Kommunikationsmodul für die Kommunikation nach außen.

Bei den Individualmodulen sind vorteilhafterweise mehrere verschiedene Individualmodule vorgesehen, die sich funktional unterscheiden und so Arten von Individualmodulen bilden. Beispiele für Arten von Individualmodulen sind Leistungsversorgungen des Elektrochirurgie-Generators, Inverter zur Erzeugung der Ausgangsenergie (Hochspannung), Anschlüsse ("Sockets", daher auch als Socket-Module bezeichnet) insbesondere Steckanschlüsse für elektrochirurgische Instrumente, und/oder Frontplatten-Module, welche typischerweise an der Frontseite angeordnete Bediener-Module meist mit Display umfassen.

Innerhalb einer Art von Individualmodulen (beispielsweise also bei der Individualmodul-Art "Inverter") können vorzugsweise verschiedene Varianten vorgesehen sein, die sich funktional unterscheiden. Die Unterscheidung kann liegen in zusätzlichen Funktionen und/oder größerer bzw. stärkerer Auslegung und Dimensionierung. So kann wahlweise bei der Individualmodule-Art "Inverter" vorgesehen sein, dass neben einer Grundversion eines Inverters ein fortgeschrittener Inverter vorgesehen ist, der zusätzliche Arten von Hochspannungserzeugung beherrscht, bspw. mittels komplexerer Kurvenformen ("Modes") oder zusätzlicher wählbarer Kurvenformen, oder eine höhere Hochspannung aufweist oder ggf. einfach nur mehr Ausgangsenergie erzeugt. Entsprechendes gilt für die Individualmodul-Art betreffend die Leistungsversorgung; innerhalb dieser Art von Modulen können Varianten mit kleinerer oder größerer Leistung vorgesehen sein, die beispielsweise mit einer festen oder einer variablen Spannung abgegeben wird zur Versorgung des Inverters und ggf. auch anderer Module des Elektrochirurgie-Generators.

Zweckmäßigerweise ist vorgesehen, dass die Individualmodule einzeln austauschbar sind. Dies stellt einen signifikanten Vorteil der Modulbauweise mit den Individualmodulen dar, da so das Erzeugen von weiteren Konfigurationen mit abweichender Ausrüstung mit Individualmodulen leicht möglich ist. Der Hersteller kann so mit wenig Aufwand viele verschiedene, an unterschiedliche Anforderungen und Anwendungsgebiete angepasste Konfigurationen herstellen.

Vorzugsweise ist weiter mindestens ein Optionalmodul mit zusätzlicher Funktionalität vorgesehen. Dieses ist wahlfrei hinzufügbar, kann also hinzugefügt werden oder auch nicht. Vorzugsweise ist es so ausgestaltet, dass im montierten Zustand selbsttätig eine Stromversorgung und Kommunikationsverbindungen mit anderen Modulen hergestellt sind. Auf diese Weise können optionale Zusatzfunktionen als Module in das erfindungsgemäße Modulsystem integriert sein. Ein Beispiel hierfür ist insbesondere ein Modul mit einem Ultraschall-Erzeuger für chirurgische Ultraschall-Instrumente oder ein Modul zur Edelgaszuführung, beispielsweise ein Argon-Modul.

Zweckmäßigerweise umfasst das modulare Generatorsystem mehrere verschiedene Baureihen von Generatortypen, für die jeweils eigene Individualmodule vorgesehen sind, die sich von den Individualmodulen einer anderen Baureihe unterscheiden. Auf diese Weise kann mittels der Individualmodule auch eine Differenzierung zwischen verschiedenen Baureihen vorgenommen werden. Dies ermöglicht es auch, nach Komplexität gestaffelte Baureihen vorzusehen. So kann eine einfache Baureihe mit einer gewissen Anzahl von Individualmodulen vorgesehen sein sowie eine gehobene Baureihe, bei der zusätzliche Individualmodule und auch zusätzliche Arten von Individualmodule vorgesehen sind bzw. montiert werden können. Auf diese Weisen ist eine feinere Ausdifferenzierung des Produktspektrums für das erfindungsgemäße Generatorsystem ermöglicht, ohne dass die Komplexität unnötig zunimmt. Weiter ist es damit ermöglicht, eine kostengünstige Einstiegsserie anzubieten, ohne am anderen Ende auf die volle Variabilität des Modulsystems zu verzichten. Besonders vorteilhaft ist es hierbei, wenn das Basismodul baureihenübergreifend einsetzbar ist. Damit wird an einer zentralen und entscheidenden Stelle eine Vereinheitlichung realisiert und damit eine solide Grundlage gelegt, auf deren Basis dann eine Differenzierung über die Individualmodule erfolgen kann.

Nun wieder Bezug nehmend auf den Basismodul-Aspekt, ist vorzugsweise vorgesehen, dass das Basismodul oder zumindest eines der Basismodule eine Stromversorgungs-Verteilungseinheit für andere Module und/oder Konnektoren für das mindestens eine Modul für den Ausgangsanschluss aufweist. So kann eine zweckmäßige Stromversorgung für die Module bereitgestellt werden, wobei zusätzlich mit dem Konnektor noch eine Aufnahmemöglichkeit für das Ausgangsanschluss-Modul geschaffen ist. Zwar wird es grundsätzlich reichen, wenn lediglich ein Konnektor vorgesehen ist, jedoch wäre dann das Basismodul begrenzt auf Generatortypen mit lediglich einem Modul für den Ausgangsanschluss. Indem es mehrere Konnektoren aufweist, bestehen somit mehrere Aufnahmeplätze für Ausgangsanschluss-Module, was auch Varianten mit einer Mehrzahl von Ausgangsanschlüssen ermöglicht, und zwar mittels desselben Basismoduls. Der oder die Konnektoren sind vorzugsweise jeweils als Steckplatz bzw. Steckplätze ausgeführt. Zweckmäßigerweise ist vorgesehen, dass an den Konnektoren Anschlüsse für ein Kommunikationsnetzwerk, ein Stromversorgungsnetz und vorzugsweise auch die Ausgangsenergieleitung aufweist. Damit wird eine vollständige Versorgung und Anbindung der an den Konnektoren angeschlossenen Individualmodule erreicht, und zwar sowohl in Bezug auf die Versorgung mit elektrischer Energie wie auch in Bezug auf die Einbindung an das Kommunikationsnetz zur Datenkommunikation mit anderen Modulen wie auch zum Anschluss an elektrische Signale. Bevorzugt ist, wenn an den Konnektoren auch die Ausgangsenergie angelegt ist, wie sie von dem Inverter insbesondere als Hochspannung erzeugt ist. Es versteht sich, dass eine solche Ausgangsenergieleitung räumlich abgesetzt und elektrisch isoliert von dem Kommunikationsnetzwerk und der Stromversorgung erfolgt, um ein unerwünschtes und für die Betriebssicherheit des Generators sowie für deren Nutzer gefährliches Überschlagen der für die Ausgangsenergie erforderlichen (mitunter hohen) Ausgangsspannung zu vermeiden und störende Einstreuung aufgrund von deren Hochfrequenz zu minimieren.

Mit Vorteil ist vorgesehen, dass die Module jeweils eine eigene Kommunikationseinheit aufweisen, die im montierten Zustand mit den Kommunikationseinheiten der anderen Module ein Kommunikationsnetzwerk zur Intermodulkommunikation bilden, das vorzugsweise selbstkonfigurierend ist. Unter der Intermodulkommunikation wird die Kommunikation zwischen den Modulen, also von einem Modul zu einem anderen verstanden. Durch die Schnittstelleneinheit ist sichergestellt, dass die Module zur eigenständigen Kommunikation mit den übrigen Modulen ausgerüstet und befähigt sind. Die Module können über das so gebildete Kommunikationsnetzwerk ihre Kommunikation zum Großteil eigenständig durchführen, so dass das Basismodul nicht zwingend mit der Bereitstellung und Durchführung der gesamten Kommunikation mit und zwischen den Modulen belastet ist. Insbesondere ist eine direkte Modul-zu-Modul Kommunikation ermöglicht, was das Basismodul entsprechend entlastet. Es ist äußerst zweckmäßig, wenn das Basismodul ebenfalls an dem Kommunikationsnetzwerk teilnimmt. Es kann mit Vorteil sogar vorgesehen sein, dass das Basismodul nicht nur an dem Kommunikationsnetzwerk teilnimmt, sondern sogar eine zentrale Aufgabe der Kommunikation übernimmt und insoweit als Master fungieren kann. Mit Vorteil ist das Kommunikationsnetzwerk selbstkonfigurierend ausgeführt. Das bedeutet, dass es zur Bildung eines Kommunikationsnetzwerks nicht zwingend der Existenz eines Masters bedarf, welcher die Konfiguration der einzelnen Teilnehmer an dem Kommunikationsnetzwerk vornimmt bzw. die Kommunikation im Kommunikationsnetzwerk überwacht und steuert.

Zweckmäßigerweise ist zumindest hinsichtlich der Individualmodul vorgesehen, dass in Bezug auf ihre Kommunikationseinheiten und ihre Stromversorgung einheitliche Schnittstellen nach au-ßen zu anderen Modulen aufweisen. Damit sind die Individualmodule nach außen hin, also zu den anderen Modulen, bezüglich ihrer Schnittstellen gleich. Ein Austausch gegen ein anderes Individualmodul, auch mit nicht identischer Funktion ist damit erleichtert, da dieses andere Individualmodul trotz seiner Andersartigkeit nach außen, also zu den anderen Modulen hin, die gleichen Schnittstellen aufweist. Allerdings können sich die Kommunikationsschnittstellen hinsichtlich ihrer Geschwindigkeit unterscheiden, wie nachfolgend erläutert ist.

Mit Vorteil ist das Kommunikationsnetzwerk so ausgeführt, dass es ein schnelleres und ein gesondertes langsameres Netzwerk umfasst. An das schnelle Kommunikationsnetzwerk sind vorzugsweise das Basismodul und all die Individualmodule angeschlossen, welche kritisch sind für den Betrieb des Elektrochirurgie-Generators. Vorzugsweise ist das schnellere Datennetzwerk als ein echtzeitfähiges Datennetzwerk ausgeführt. An das langsamere Netzwerk sind vorzugsweise solche Module angeordnet, die nicht bzw. nur weniger kritisch sind für den Betrieb des Elektrochirurgie-Generators oder die wiederum nur einen geringen Umfang an Datenverkehr benötigen, der nicht zeitkritisch ist. Das langsamere Datennetzwerk eignet sich auch zur Verbindung von Untereinheiten mit Modulen bzw. bei Modulen, die in zwei oder mehr räumlich getrennte Einheiten geteilt sind, zu deren Kommunikation untereinander. Zweckmäßig ist es, wenn das schnellere Datennetzwerk als CAN-Netzwerk ausgeführt ist. Dieses ermöglicht eine hohe Kommunikation mit der Fähigkeit zur Echtzeit. Auch das langsamere Datennetzwerk kann als ein (langsameres) CAN-Netzwerk ausgeführt sein.

Für das Basismodul kann optional eine Erweiterungseinheit vorgesehen sein. Diese Erweiterungseinheit weist zweckmäßigerweise zusätzliche Konnektoren auf. Damit können die notwendigen Voraussetzungen geschaffen werden um an den zusätzlichen Konnektoren zusätzliche Module aufzunehmen, um so insbesondere den Elektrochirurgie-Generator mit zusätzlichen Ausgangsanschluss-Modulen ausrüsten zu können. Dies ist insbesondere für höherwertig ausgestattete Generatortypen von Vorteil, bei der eine Vielzahl von Ausgangsanschlüssen wie monopolare Sockets, bipolare Sockets, universelles Sockets und gegebenenfalls auch sog. Neutralsockets vorgesehen sind. Gerade bei den Sockets kommt der Vorteil des Basismoduls mit seiner Erweiterungseinheit voll zum Tragen, da hier über das Basismodul ggf. auch die (meist hohe) Spannung für die Ausgangsenergie direkt zugeführt werden kann, und somit kein zusätzlicher Aufwand bzw. schwierig zu isolierende Verdrahtung in dem Elektrochirurgie-Generator anfällt. Vorzugsweise weist dazu die Erweiterungseinheit auch eine Ausgangsenergieleitung auf, die - genauso wie am Basismodul - hochspannungsführend für die Ausgangsenergie ausgebildet ist.

Es kann, wie bereits erwähnt, das Basismodul auf unterschiedliche Weise ausgeführt sein. Einer Ausführung des Basismoduls als Displayansteuerungsmodul liegt der Gedanke zugrunde, dass so vorteilhafterweise eine Trennung von Displayanzeige einerseits und dem Displayansteuerungsmodul andererseits ermöglicht ist. Somit kann das Displayansteuerungsmodul als Basismodul ausgeführt sein, während je nach Ausstattung ein kleineres oder größeres Display als Anzeige verbaut sein kann. Nach au-ßen zum Nutzer hin ist so eine Differenzierung erreicht, während auf der technischen Seite das für die Ansteuerung des Displays verwendete Modul (Displayansteuerungsmodul) als Basismodul identisch bleibt, was den Aufbau sowie die Einbindung in das Kommunikationsnetzwerk und die Konfiguration vereinfacht.

Die Erfindung erstreckt sich ferner auf ein modulares Elektrochirurgie-Generatorsystem umfassend wenigstens zwei Generatortypen von Elektrochirurgie-Generatoren, die sich bezüglich ihrer Funktionen unterscheiden, wobei die Generatortypen jeweils zur Speisung eines elektrochirurgischen Instruments ausgebildet sind und zusammengesetzt sind aus mehreren Generatorkomponenten umfassend eine Leistungsversorgung und einen Inverter, gespeist von der Leistungsversorgung zur Erzeugung einer Ausgangsenergie, die zu mindestens einem Ausgangsanschluss zum Anschluss des elektrochirurgischen Instruments geführt ist, wobei erfindungsgemäß das modulare Generatorsystem umfasst (i) eine Anzahl verschiedener Invertermodule, woraus eines für den jeweiligen Generatortyp ausgewählt und montiert ist, und (ii) eine Anzahl verschiedener Ausgangsanschlüsse, von denen mindestens einer ausgewählt und in eine Frontplatte mechanisch aufgenommen sowie mit dem montierten Inverter elektrisch verbunden ist, wobei jeder Elektrochirurgie-Generator aufweist einen Modul-Detektor, der zur Erkennung montierter und angeschlossener Module ausgebildet ist und daraus eine real vorhandene Modulkonfiguration ermittelt. Die Frontplatte kann hierbei ebenfalls als Modul ausgeführt sein (Frontplattenmodul), zwingend ist das jedoch nicht.

Dieser Erfindungsaspekt basiert auf dem Gedanken, dass eine Anzahl verschiedener Module existiert, zu denen mindestens verschiedene Invertermodule gehören sowie verschiedene Ausgangsanschlüsse, wobei jeweils mindestens einer ausgewählt und entsprechend montiert ist. Ferner können weitere Module vorgesehen sein, wie sie insbesondere vorstehend beschrieben sind als Basis- und/oder Individualmodule. Auf diese Weise ist ein sehr variabler Aufbau bzw. Bestückung des Elektrochirurgie-Generators mit Modulen ermöglicht. Für den Betrieb des Elektrochirurgie-Generators ist es jedoch entscheidend, dass Klarheit herrscht über die vorhandene Ausstattung mit Modulen (Modulkonfiguration). Zu diesem Zweck ist ein Modul-Detektor vorgesehen, der zur Erkennung montierter und angeschlossener Module ausgebildet ist und daraus die real vorhandene Modulkonfiguration ermittelt. Dies erfolgt zweckmäßigerweise automatisiert. Damit kann bei der Montage im Grunde frei aus den Modulen je nach den spezifischen Anforderungen des Kunden oder aufgrund von Baureihenanforderungen des Herstellers ausgewählt werden, wobei der Modul-Detektor bei Inbetriebnahme bzw. im Betrieb erfasst, welche Module tatsächlich vorhanden sind und in welcher Konfiguration sie zum Betrieb des Elektrochirurgie-Generators genutzt werden können. Somit ergeben sich eine Selbstkontrolle und eine Selbstüberwachung.

Es versteht sich, dass die Modulerkennung nur in Bezug auf kooperierende Module wirken kann, die über entsprechende Mittel für eine elektronische Erkennung verfügen. Passive Bauelemente ohne solche elektronischen Mittel, wie bspw. das Gehäuse, können und brauchen nicht detektiert zu werden.

Ferner kann dies mit Vorteil genutzt werden für eine Selbstkonfiguration. Dazu weisen die Module vorzugsweise eine eigene Selbstkonfigurationseinheit auf, die dazu ausgebildet ist, das jeweilige Modul abhängig von der ermittelten real vorhandenen Modulausrüstung zu konfigurieren. Unter der real vorhandenen Modulausrüstung werden zum einen die Module verstanden, die tatsächlich physisch vorhanden ist, zum anderen aber auch der Aspekt, wie weit das Modul jeweils aktiviert ist. Letzteres ist insbesondere von Bedeutung bei Modulen, die in Bezug auf ihre Hardware gleich oder ähnlich sind, aber bei denen unterschiedliche Firmware vorhanden ist bzw. die Firmware zu einem unterschiedlichen Grad freigeschaltet ist, um so unterschiedliche Funktionalitäten (in der Regel einen kleinen und einen größeren Funktionssatz) zu ermöglichen und gezielt abrufbar zu machen. Die Selbstkonfigurationseinheit ermittelt dies und stellt entsprechend die Konfiguration ihres Moduls selbsttätig ein. Es wird damit erreicht, dass die Konfiguration der Module stets mit der tatsächlich vorhandenen und aktivierten Modulausrüstung übereinstimmen bzw. dazu passt. Die Gefahr von Fehlkonfigurationen und dadurch entstehende Betriebsstörungen bzw. die Gefahr einer Beschädigung des Geräts oder Schädigung des Patienten werden dadurch minimiert.

Der Modul-Detektor ist zweckmäßigerweise als eine zentrale Einheit für den jeweiligen Elektrochirurgie-Generator ausgeführt. Dies hat den Vorteil, dass Information über die Modulausrüstung an einer zentralen Stelle ermittelt ist und dort abrufbar ist. Der Gefahr von Inkonsistenzen bei der Konfiguration der einzelnen Module wird damit entgegengewirkt. Mit Vorteil ist der Modul-Detektor angeordnet auf dem Basismodul oder einem der Basismodule. Zwingend ist dies jedoch nicht.

Es kann aber auch vorgesehen sein, dass der Modul-Detektor dezentral ausgeführt ist, und zwar vorzugsweise auf den jeweiligen Modulen. Damit können die einzelnen Module jeweils für sich selbst bestimmen, in welcher Modul-Umgebung sie eingesetzt sind, und basierend auf dieser Information ist dann die jeweilige Konfiguration der Module.

Mit Vorteil ist ferner eine Freischalt-Einheit vorgesehen, die dazu ausgebildet ist abhängig von der real vorhandenen Modulkonfiguration freigegebene Funktionen zu ermitteln und entsprechende Freischaltsignale an die Module zu übermitteln. Auf diese Weise kann die Funktionalität der einzelnen Module und/oder der Umfang der Funktionalität bestimmt und eingestellt werden abhängig von der real vorhandenen Modulkonfiguration. Damit ist sichergestellt, dass jedes Modul so konfiguriert ist, dass es zu dem jeweiligen Elektrochirurgie-Generator und den dort verbauten Modulen passt. Die Gefahr von Fehlkonfigurationen, wie sie typischerweise bei herkömmlicher Konfigurationsweise auftreten konnten, wird damit wirksam verhindert.

Hierbei kann die Freischalt-Einheit als eine zentrale Einheit ausgeführt sein, welche die entsprechende Freischaltung der Module zentral übernimmt. Es kann aber auch vorgesehen sein, dass die Freischalt-Einheit dezentral auf den jeweiligen Modulen ausgeführt ist. Vor- und Nachteile entsprechen sinngemäß denjenigen, wie die vorstehend zur zentralen bzw. dezentralen Ausführung des Modul-Detektors angeführt sind.

Zweckmäßigerweise ist vorgesehen, dass die einzelnen Module dazu ausgebildet sind, dass im montierten Zustand selbsttätig Kommunikationsverbindungen und eine Stromversorgung der Module hergestellt sind. Auf diese Weise ist über die eigentliche Montage der Module hinaus keine eigenständige Tätigkeit für die Module in Bezug auf Herstellung der Kommunikationsverbindungen bzw. deren Stromversorgung erforderlich. Dies vereinfacht das Einsetzen der Module und sowie die Inbetriebnahme bzw. anfängliche Konfiguration. Durch die selbsttätige Ausführung sind Fehlerquellen, wie sie durch händisches Eingreifen entstehen können, praktisch ausgeschlossen.

Bei den Kommunikationsverbindungen handelt es sich meist um ein Datennetzwerk, wobei vorzugsweise zusätzliche Signalverbindungen vorgesehen sind für Freigabesignale bezüglich Aktivität der Module. Indem die Module sich vorzugsweise selbstständig auf die Kommunikationsverbindungen konfigurieren, ist auf diese Weise automatisch deren Teilnahme am Datennetzwerk und somit dem Datenaustausch zu den anderen Modulen, wie dem Basismodul und oder Individualmodulen, ohne weiteres Zutun sichergestellt. Dies vereinfacht die Konfiguration und verringert die Gefahr von Fehlerquellen. Mit den Freigabesignalen kann ferner gesteuert werden, ob gewisse Module aktiv sind bzw. aktiviert werden und so überhaupt teilnehmen. Das ermöglicht eine einfache Differenzierung der Funktionsausstattung, indem gewisse Module oder Funktionalitäten der Module freigegeben bzw. nicht freigegeben werden. Somit kann das Modulsystem bezüglich seiner Granularität verfeinert werden, ohne dass dazu zusätzliche weitere Module erforderlich sind. Außerdem ergibt sich ein zusätzlicher Sicherheitsaspekt, indem als nicht dazugehörend oder als defekt erkannte Module nicht freigeschaltet werden und somit aus dem Betrieb des Elektrochirurgie-Generators herausgehalten werden.

Mit Vorteil weisen die Module jeweils eine eigene Prozessoreinheit mit einem eigenen Steuerprogramm als eigenständige lokale Betriebssteuerung für das jeweilige Modul auf, und sie sind angeschlossen an ein generatorinternes Stromversorgungsnetz sowie Kommunikationsnetzwerk. Mit dem eigenen Prozessor und Steuerprogramm sind die Module insoweit autark und bedürfen keiner Steuerung von modul-extern, beispielsweise von einer zentralen Einheit. Indem die Module dies autark für sich erledigen, werden so der Datenverkehr reduziert und die Verarbeitungsgeschwindigkeit bzw. -geschwindigkeit erhöht. Außerdem kann auf diese Weise je nach Modul bzw. je nach Modulkomplexität ein passender Prozessor vorgesehen sein, so dass dessen Leistungsfähigkeit stets angepasst ist an die Anforderungen des jeweiligen Moduls.

Zumindest die Hochspannungserzeugungsmodule und/oder optionale Ultraschallmodule weisen einen zusätzlichen Hochfrequenz-Ausgangsanschluss auf. Mit einem solchen zusätzlichen Anschluss für diese besondere Ausgangsenergie (Hochspannung bzw. Ultraschall) sind die entsprechenden Module auch autark hinsichtlich des Ausgangs. Damit werden die Anschlusswege für diese besondere Ausgangsenergie verkürzt und ggf. auch ferngehalten von den übrigen Modulen. Ferner eignen sich diese Module insbesondere auch zur Nachrüstung bestehender Elektrochirurgie-Generatoren, und zwar auch von solchen, deren Ausgangsanschlüsse bereits anderweitig ausgeschöpft sind.

Weiter sind zweckmäßigerweise eine Anzahl verschiedener Leistungsversorgungsmodule vorgesehen, woraus eines für den jeweiligen Generatortyp ausgewählt und montiert ist. Auf diese Weise kann ähnlich wie bei den bereits erwähnten Invertermodulen eine an die Größe und den Ausstattungsumfang des jeweiligen Elektrochirurgie-Generators angepasste Leistungsversorgung auf einfache Weise ausgewählt und bereitgestellt werden. Durch die Einbeziehung der Leistungsversorgung in das modulare Konzept wird eine erhebliche Steigerung der Bandbreite der Modularisierung erreicht.

Bei einer besonders zweckmäßigen Ausgestaltung, die gegebenenfalls unabhängigen Schutz verdient, ist eine Sicherungs-Einheit vorgesehen, die dazu ausgebildet ist eine real vorhandene Modulkonfiguration mit einem eindeutigen, fest eingespeicherten Sollausrüstungsmerkmal für die Modulkonfiguration zu vergleichen und bei Abweichung ein Fehlersignal auszugeben, das vorzugsweise eine Betriebssperre betätigt. Bei dem fest eingespeicherten Sollausrüstungsmerkmal handelt sich um einen Datensatz, der typischerweise vom Hersteller selbst festgelegt und fest eingespeichert wird. Dieser Datensatz steht für die vom Hersteller vorgesehene Konfiguration der Module. Später wird bei Inbetriebnahme bzw. beim Kunden im Rahmen des üblichen Betriebs von der Sicherungseinheit des Elektrochirurgie-Generators die real vorhandene Modulkonfiguration herangezogen (wie sie typischerweise vom Modul-Detektor bestimmt ist) und mit dem eingespeicherten Sollausrüstungsmerkmal verglichen. Bei Übereinstimmung der real vorhandenen Modulkonfiguration mit der gemäß dem Sollausrüstungsmerkmal wird der Betrieb freigegeben. Besteht keine Übereinstimmung, so liegt entweder ein Defekt des Elektrochirurgie-Generators bzw. eines seiner Module vor oder es wurde an den Modulen bzw. ihrer Konfiguration manipuliert; dann wird das Fehlersignal ausgegeben und in der Folge mit Vorteil der Betrieb des Elektrochirurgie-Generators zur Sicherheit gesperrt. Gegebenenfalls kann vorgesehen sein, dass die Sperre nur teilweise ist, sodass sofern möglich noch ein Grundbetrieb weiterhin stattfinden kann.

Mit Vorteil ist ferner eine Upgrade-Einheit vorgesehen, die von extern über eine gesicherte Kommunikationsverbindung ansteuerbar ist und mit der Sicherungs-Einheit und der Selbstkonfigurationseinheit der Module zusammenwirkt, um das eingespeicherte Sollausrüstungskennungsmerkmal mit einer autorisierten Änderung zu versehen und/oder zusätzliche Funktionen freizuschalten. Auf diese Weise können in einer autorisierten Weise Anpassungen hinsichtlich der Modulkonfiguration vorgenommen werden, ferner können zusätzliche Funktionen freigeschaltet werden. Dies ermöglicht es, auch nach der Herstellung des Elektrochirurgie-Generators zusätzliche Module einzubinden und zu nutzen bzw. die Funktionalität vorhandener Module gegebenenfalls zu erweitern. Die gesicherte Kommunikationsverbindung kann ein gesicherter Fernzugang sein. So wird erreicht, dass die Anpassung der Modulkonfiguration auch aus der Ferne erfolgen kann, beispielsweise von dem Hersteller des Elektrochirurgie-Generators. Dies kann im Rahmen einer Aktualisierung, beispielsweise der Firmware der Module, geschehen oder im Rahmen eines Upgrade, womit zusätzliche Funktionen freigegeben und/oder zusätzlich eingebaute Module freigeschaltet werden.

Die Erfindung wird nachfolgend unter Bezugnahme auf die beigefügte Zeichnung beispielhaft anhand einer vorteilhafter Ausführungsformen erläutert. Es zeigen:
- Fig. 1: eine schematische Darstellung eines Elektrochirurgie-Generators gemäß einem Ausführungsbeispiel mit einem angeschlossenen elektrochirurgischen Instrument;
- Fig. 2: ein Blockdiagramm zu dem Elektrochirurgie-Generators gemäß Fig. 1;
- Fig. 3: eine schematische Darstellung für weitere Funktionseinheiten am Beispiel eines Basismoduls;
- Fig. 4a-c: schematische Darstellungen zur Anordnung der Module in verschiedenen Gehäusen; und
- Fig. 5a-c: perspektivische Darstellungen verschiedener Ausführungsbeispiele zur Anordnung der Module in verschiedenen Gehäusen.

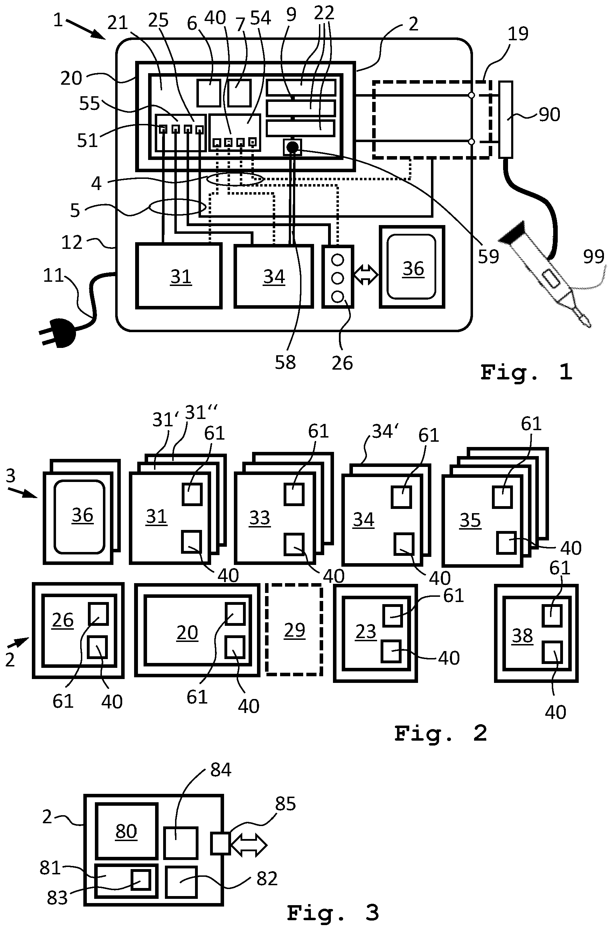

Ein Elektrochirurgie-Generator gemäß einem Ausführungsbeispiel der Erfindung ist in Figur 1 dargestellt. Der in seiner Gesamtheit mit der Bezugsziffer 1 bezeichnete Elektrochirurgie-Generator umfasst ein Gehäuse 12, das mit einem Ausgangsanschluss 19 für ein elektrochirurgisches Instrument 99 versehen ist, in dem dargestellten Ausführungsbeispiel handelt es sich hierbei um ein elektrisches Skalpell. Es ist über einen Anschlussstecker 90 eines Hochvoltverbindungskabels 98 mit einem Ausgangsanschluss 19 des Elektrochirurgie-Generators 1 verbunden.

Der Elektrochirurgie-Generator 1 ist mit einem Netzanschlusskabel 11 versehen, über das er mit dem öffentlichen Stromnetz verbunden und daraus gespeist werden kann. Es sei angemerkt, dass zur Speisung auch andere Quellen verwendet werden können, beispielsweise eine Gleichspannungsspeisung mit 12 V oder 48 V bei Elektrochirurgie-Generatoren 1, die in Fahrzeugen oder in anderen Umgebung mit hauptsächlich Gleichspannungsversorgung eingesetzt werden. Das Netzanschlusskabel 11 (oder eine andere Art der Speisung, wie die Gleichspannungsspeisung) ist angelegt an ein Leistungsversorgungsmodul 31, welches daraus die für den Betrieb der Komponenten und Module des Elektrochirurgie-Generators 1 benötigten Spannungen bereitstellt und über ein Stromversorgungsnetz 5 an die einzelnen Module verteilt. So wird darüber von der Leistungsversorgung 31 ein Inverter 34 zur Erzeugung der an das elektrochirurgische Instrument 99 abzugebenden Ausgangsenergie (Hochspannung) gespeist.

Der Inverter 34 erzeugt aus der vom Leistungsmodul 31 zugeführten Energie eine hochfrequente Wechselspannung als Ausgangsenergie. Sie liegt typischerweise im Hochspannungsbereich, kann aber Amplituden im Bereich von einigen 10 V bis 4000 V aufweisen. Die Art der Ausführung des Inverters 34 zur Erzeugung der Ausgangsenergie ist insoweit offen, als an sich beliebige der bekannten Konzepte, beispielsweise Eintaktwandler oder Inverter im engeren Sinne, vorgesehen sein können. Wesentlich ist, dass dieser die für das elektrochirurgische Instrument 99 benötigte Ausgangsenergie in Bezug auf hohe Frequenz und ebenfalls hohe Spannung erzeugt. Diese so erzeugte Ausgangsenergie wird über das Verteilungsmodul 20 mittels einer Ausgangsenergie-Leitung 9 zu dem Ausgangsanschluss 19 zum Anschluss des elektrochirurgischen Instruments 99 geführt.

Zur Einstellung der Betriebsweise und zur Anzeige gewisser Funktionen ist ein Displayansteuerungsmodul 26 vorgesehen, welches im Bild symbolhaft dargestellt ist mit einem Bedienungsknöpfe aufweisenden Nutzer-Interface, über das der Nutzer den Elektrochirurgie-Generator 1 nach seinen Wünschen einstellen kann. Es wirkt zusammen mit einer Anzeige, die als Displaymodul 36 ausgeführt ist.

Insoweit sind die vorzusehenden Komponenten und deren Funktionen im Grunde bekannt. Die Besonderheit der Erfindung liegt insbesondere darin, dass Komponenten des Elektrochirurgie-Generators in Modulbauweise ausgeführt sind. Dazu wird Bezug genommen auf Figur 2. Dort sind Beispiele für verschiedene Module dargestellt, aus denen ausgewählt werden kann zur Schaffung von sich in Ausstattung und Funktion unterscheidenden Generatortypen von Elektrochirurgie-Generatoren 1. In der unteren Zeile sind Basismodule 2 dargestellt, von denen mindestens eines vorhanden sein muss und das typischerweise über verschiedene elektrochirurgische Generatoren 1 einer Baureihe identisch ist.

Zu den Basismodulen 2 zählt insbesondere ein Verteilungsmodul 20, welches eine Stromversorgungs-Verteilungseinheit 25 sowie eine Kommunikationseinheit 40 aufweist und sowohl die für den Betrieb erforderliche elektrische Leistung an die übrigen Module verteilt und entsprechend mit den anderen Modulen über die Kommunikationseinheit 40 und ein daran angeschlossenes Kommunikationsnetzwerk 4 kommuniziert. Häufig wird es genügen, wenn ein einziges Basismodul 2 in Gestalt des Verteilungsmoduls 20 vorgesehen ist. Allerdings ist dies nicht zwingend, es kann auch vorgesehen sein, dass besonders aufwändige Generatoren 1 vorzugsweise aus einer anderen Baureihe mit einem anderen Basismodul 2 versehen sind. Weitere Basismodule können insbesondere sein das Displayansteuerungsmodul 26 oder ein zur Kommunikation vorgesehenes Rückseitenmodul 27 oder ein ebenfalls zur Kommunikation eingerichtetes Frontseitenmodul 23. Optional kann weiter für das Verteilungsmodul 20 eine Erweiterungseinheit 29 vorgesehen sein, die lediglich mit dem Verteilungsmodul 20 zu koppeln ist, um so weitere Anschlussplätze für zusätzliche Module des Elektrochirurgie-Generators 1 bereitzustellen. Auf diese Weise können auch Generatortypen mit sehr umfangreicher Ausrüstung und vielen Individualmodulen 3 unter Verwendung von demselben Basismodul 2 aufgebaut sein.

In der oberen Zeile von Figur 2 dargestellt sind Individualmodule, die typischerweise zum Betrieb des Elektrochirurgie-Generators 1 ebenfalls benötigt werden, bei denen aber Auswahl besteht. Auf diese Weise können durch Wahl unterschiedlicher Individualmodule 3 verschiedene Generatortypen geschaffen werden, die sich in Bezug auf Ausstattung und Funktion unterscheiden. Zu den Individualmodulen gehören insbesondere neben dem Leistungsversorgungsmodul 31 und dem Inverter 34 ein Frontplattenmodul 32, mindestens ein Anschlussmodul 35 für die Anschlussbuchse 19, das Displaymodul 36.

Das Basismodul 2 sowie die Individualmodule 3 sind jeweils mit einer Kommunikationseinheit 40 versehen. Über diese ist das jeweilige Modul funktional mit dem Kommunikationsnetzwerk 4 verbunden. Das Kommunikationsnetzwerk 4 ist als CAN-Netzwerk ausgeführt, und zwar zweigeteilt mit einem schnelleren und einem langsameren Netzwerk.

Es wird nun wieder Bezug genommen auf Fig. 1, wo ein Verteilungsmodul 20 als Basismodul 2 dargestellt ist. Es umfasst eine Stromversorgungs-Verteilungseinheit 25 sowie eine Kommunikationseinheit 40 auf einer optionalen Grundplatte 21. Ferner sind Anschlussstecker 54, 55 an dem Verteilungsmodul 20 vorgesehen für die Verbindung zwischen dem Basismodul 2 und den Individualmodulen 3.

Über einen Versorgungstecker 51 ist das Leistungsversorgungsmodul 31 angeschlossen. Über diesen wird die von dem Leistungsversorgungsmodul 31 bereitgestellte Leistung zu dem Verteilungsmodul 20 geführt, welches die Leistung dann an die Basis- und Individualmodule 2, 3 über die Stromversorgungs-Verteilungseinheit 25 weiterleitet.

Der Inverter 34 erzeugt in an sich bekannter Weise aus der von der Leistungsversorgung 31 zugeführten elektrischen Leistung eine Wechselspannung, die als Ausgangsenergie an das chirurgische Instrument 99 abzugeben ist. Dazu ist die von dem Inverter 34 abgegebenen Wechselspannung, die typischerweise eine Hochspannung ist, über eine Hochspannungsleitungsverbindung 58 geführt zu dem Verteilungsmodul 20, und zwar an einen dort angeordneten Hochspannungsanschluss 59. Von dem Hochspannungsanschluss 59 ist die Hochspannung über eine in das Verteilungsmodul 20 integrierte Ausgangsenergieleitung 9 angelegt. Diese ist u.a. geführt zu einer Mehrzahl von Konnektoren 22 auf dem Verteilungsmodul 20, wobei in die als Aufnahmeplätze ausgeführten Konnektoren 22 jeweils ein Anschlussmodul 35 für das elektrochirurgische Instrument 99 aufnehmbar ist.

Auf diese Weise kann mindestens ein und optional mehrere Anschlussmodule 35 mit der vom Inverter 34 erzeugten Hochspannung versorgt werden, nämlich einfach in dem diese Anschlussmodule 35 in ihren jeweiligen Konnektor 22 eingesetzt sind. Weist bspw. ein Elektrochirurgie-Generator 1 eines anderen Generatortyps zusätzliche Ausgangsanschlüsse 19` auf, so sind dazu lediglich zusätzliche Anschlussmodule 35 vorzusehen, welche einfach jeweils in einen der Konnektoren 22 einzusetzen sind und dann automatisch versorgt sind, und zwar auch mit der vom Inverter 34 erzeugten Hochspannung. Auf diese Weise können auch komplexe Elektrochirurgie-Generatoren 1 mit einer Mehrzahl von verschiedenen Anschlüssen 19 auf einfache Weise modular realisiert sein, nämlich indem einfach entsprechend viele oder verschiedene Anschlussmodule 35 in einen oder mehrere der vorgesehenen Konnektoren 22 eingesteckt sind.

Es versteht sich, dass die Konnektoren 22 ebenso angeschlossen sind an die Stromversorgungs-Verteilungseinheit 25 und Kommunikationseinheit 40, um entsprechend versorgt zu sein (in Figur 1 nicht dargestellt).

Es ist somit eine Dreifachversorgung, nämlich für die reguläre Stromversorgung, die Datenkommunikations-Verbindung sowie für die Hochspannung als Ausgangsenergie, gebildet in Bezug auf unterschiedliche Arten von anzuschließenden Individualmodulen 3 einschließlich den Anschlussmodulen 35. Letztere sind in dem beschriebenen Ausführungsbeispiel direkt in das Verteilungsmodul 20 einzusetzen und sind dann unmittelbar mit der als Hochspannung geführten Ausgangsenergie in der Ausgangsenergie-Leitung 9 verbunden sowie an die Stromversorgungs-Verteilungseinheit 25 und Kommunikationseinheit 40 angeschlossen. Die übrigen Individualmodule 3 sind typischerweise über Kabelverbindungen an die Stromversorgungs-Verteilungseinheit 25 und Kommunikationseinheit 40 angeschlossen. Dazu sind sie über das Kommunikationsnetzwerk 4 mit der Kommunikationseinheit 40 bzw. über Anschlussstecker 55 mit der Stromversorgungs-Verteilungseinheit 25 des Verteilungsmoduls 20 verbunden. Dies sichert eine hohe Flexibilität insbesondere hinsichtlich der räumlichen Anordnung der Individualmodule 3, wie dies u.a. in Bezug auf Gehäuse 12 in verschiedenen Größen und der damit variierenden Einbaulage bzw. Abständen von Vorteil ist.

Der Elektrochirurgie-Generator 1 weist ferner einen Moduldetektor 6 auf. Dieser ist dazu ausgebildet, die in dem Elektrochirurgie-Generator 1 montierten und angeschlossenen Module und deren Konfiguration zu ermitteln. Der Moduldetektor 6 kann dezentral angeordnet sein auf den jeweiligen Modulen 2, 3. Bei dem dargestellten Ausführungsbeispiel ist der Moduldetektor 6 jedoch zentral angeordnet, und zwar an dem Verteilungsmodul 20 des Basismoduls 2. Der Moduldetektor 6 ist über (in Fig. 1 nicht dargestellte) Leitungen mit dem Stromversorgungsnetz 5 sowie dem Kommunikationsnetzwerk 4 verbunden. Der Moduldetektor 6 ist automatisiert. Über das Kommunikationsnetzwerk 4 kommuniziert der Moduldetektor 6 mit den verschiedenen Modulen 2, 3 des Elektrochirurgie-Generators 1. Dabei ermittelt der Moduldetektor 6, welche Module 2, 3 montiert und aktiv sind. Der Moduldetektor 6 bestimmt so, welche Module 2, 3 tatsächlich vorhanden sind und wie diese Module 2, 3 konfiguriert sind. Auf diese Weise erkennt der Moduldetektor 6 die Modulausstattung des Elektrochirurgie-Generators 1. Genutzt werden kann dies beispielsweise für die Erkennung der Ist-Ausstattung des Elektrochirurgie-Generators 1.

Der Moduldetektor 6 wirkt ferner mit einer Selbstkonfigurationseinheit 61 auf den Modulen 2, 3 zusammen. Dazu weisen die Module 2, 3 jeweils eine Selbstkonfigurationseinheit 61 auf. Mittels der Selbstkonfigurationseinheit 61 kann sich das jeweilige Modul 2, 3 selbsttätig konfigurieren, und zwar in Abhängigkeit von der jeweils ermittelten Ist-Ausstattung, also der real vorhandenen Modulausstattung des Elektrochirurgie-Generators 1. So kann beispielsweise das Modul 34 für den Inverter, der die Hochspannung für das elektrochirurgische Instrument 99 erzeugt, konfiguriert werden abhängig von der Art des Ausgangsanschluss 19 und dessen Ausgangsmodul 35. So können die Ausgangsenergie oder die Art der Abgabe der Ausgangsspannung, insbesondere deren Kurvenform, mithin also die "Modes" des Inverters 34 selbsttätig konfiguriert sein abhängig von dem zu versorgenden Ausgangsanschluss 19 und dessen Anschlussmodul 35. Das den Inverter 34 speisende Modul für die Leistungsversorgung 31 wird dann wiederum von seiner eigenen Selbstkonfigurationseinheit 61 bezüglich der abzugebenden Spannung bzw. Energie konfiguriert abhängig von der - wie vorstehend beschrieben - eingestellten Konfiguration des Inverters 34. So kann auf diese Weise eine praktisch vollständige Selbstkonfiguration der Module 2, 3 des Elektrochirurgie-Generators 1 erreicht werden. Dank der dezentral angeordneten Selbstkonfigurationseinheit 61 ist dies auch dann möglich, wenn Module 2, 3 ausgetauscht werden oder ein neues Individualmodul 3 hinzugefügt wird.

Ferner ist eine Freischalt-Einheit 7 vorgesehen. Sie ist in dem dargestellten Ausführungsbeispiel zentral angeordnet, und zwar an dem Verteilungsmodul 20. Die Freischalt-Einheit 7 ist dazu ausgebildet, abhängig von der (bspw. von dem Moduldetektor 6 bestimmten) real vorhandenen Modulkonfiguration freigegebene Funktionen zu ermitteln und entsprechende Freischaltsignale an die Module 2, 3 zu übermitteln. Auf diese Weise kann die Funktionalität der einzelnen Module 2, 3 insgesamt aktiviert bzw. gesperrt werden, und/oder der Umfang von deren jeweiliger Funktionalität bestimmt und eingestellt werden, und zwar abhängig von der real vorhandenen Modulkonfiguration. Dazu übermittelt die Freischalt-Einheit 7 entsprechende Freischaltsignale über das Kommunikationsnetzwerk 4 an die Module 2, 3. Damit ist sichergestellt, dass nur solche Module 2, 3 bzw. deren Funktionalität freigeschaltet und aktiviert werden, die zu dem jeweiligen Elektrochirurgie-Generator 1 und der Konfiguration mit den dort verbauten Modulen 2, 3 passen.

Es wird nun Bezug genommen auf Figur 3, welche schematisch weitere Funktionseinheiten zeigt, die an den Modulen 2, 3 angeordnet sein können. Beispielhaft ist dies in Figur 3 an dem Basismodul 2 erläutert; für die Individualmodule 3 gilt dies entsprechend. Das Basismodul 2 umfasst weiter eine Prozessoreinheit 80, die mit einem eigenen Steuerprogramm versehen ist. Dieses ist in einem Speicher 81 eingespeichert. Somit verfügt das Basismodul 2 über eine eigenständige lokale Betriebssteuerung, die wie das Basismodul 2 an das generatorinterne Stromversorgungsnetz 5 sowie Kommunikationsnetzwerk 4 angeschlossen ist. Für die ebenso ausgerüsteten Individualmodule 3 gilt dies entsprechend. Mit der eigenen Prozessoreinheit 80 sowie dem eingespeicherten Steuerprogramm sind die Module 2, 3 insoweit autark und bedürfen keiner externen Steuerung (wobei der Begriff "extern" auf das jeweilige Modul bezogen ist, im Sinne von "extern von dem jeweiligen Modul").

Ferner können weitere Funktionseinheiten vorgesehen sein, die lokal auf einem (zentral) oder mehreren der Module 2, 3 (dezentral) angeordnet sein können. In Figur 3 ist dies exemplarisch anhand des Basismoduls 2 gezeigt; für die Individualmodule 3 kann entsprechendes gelten. Demnach ist weiter vorgesehen eine Sicherungs-Einheit 82 hinsichtlich der Modulkonfiguration. Die Sicherungs-Einheit 82 ist dazu ausgebildet, eine real vorhandene Modulkonfiguration, wie sie beispielsweise von dem Moduldetektor 6 ermittelt ist, zu vergleichen mit einem Sollausrüstungsmerkmal 83. Bei dem Sollausrüstungsmerkmal 83 handelt es sich um einen Datensatz für die vom Hersteller vorgesehene Konfiguration der Module 2, 3. Bei der Inbetriebnahme bzw. beim Nutzer im Rahmen des üblichen Betriebs gleicht die Sicherung-Einheit 82 die real vorhandene Modulkonfiguration, wie sie insbesondere von dem Moduldetektor 6 ermittelt ist, ab mit dem eingespeicherten Sollausrüstungsmerkmal 83. Bei Übereinstimmung ist die Konfiguration des Elektrochirurgie-Generators 1 in Ordnung und der Betrieb kann wie vorgesehen erfolgen. Ergibt sich jedoch eine Diskrepanz, so liegt entweder ein Defekt eines der Module 2, 3 oder eine Fehlkonfiguration von mindestens einem der Module 2, 3 vor. Es wird ein entsprechendes Fehlersignal ausgegeben, vorzugsweise über das Datenkommunikationsnetz 4 und dem Nutzer auf dem Display 36 angezeigt, und in der Folge wird der Betrieb des Elektrochirurgie-Generators 1 zur Sicherheit gesperrt. Diese Sperrung kann je nach Diskrepanz vollständig oder teilweise sein, sodass ggf. bestimmte Funktionen gesperrt sind und der Elektrochirurgie-Generator 1 mit den nicht betroffenen Funktionen bzw. seinen Grundfunktionen weiterbetrieben werden kann. All dies erfolgt automatisiert durch die Sicherungs-Einheit 82.

Weiter kann eine Upgrade-Einheit 84 vorgesehen sein, die mit einer gesicherten Kommunikationsverbindung 85 zusammenwirkt. Über diese kann die Upgrade-Einheit mit der Außenwelt kommunizieren, und insbesondere von einer externen autorisierten Stelle (beispielsweise dem Hersteller) angesteuert werden. Die Upgrade-Einheit wirkt zusammen mit der Sicherungs-Einheit 82 und dem Speicher 81, um das Sollausrüstungsmerkmal 83 ggf. mit einer autorisierten Änderung zu versehen. Auf diese Weise können zusätzliche Funktionen freigeschaltet werden oder weitere zusätzliche Module 3, die nachträglich eingebaut worden sind, für den Betrieb des Elektrochirurgie-Generators 1 zugelassen und in diesen eingebunden werden.

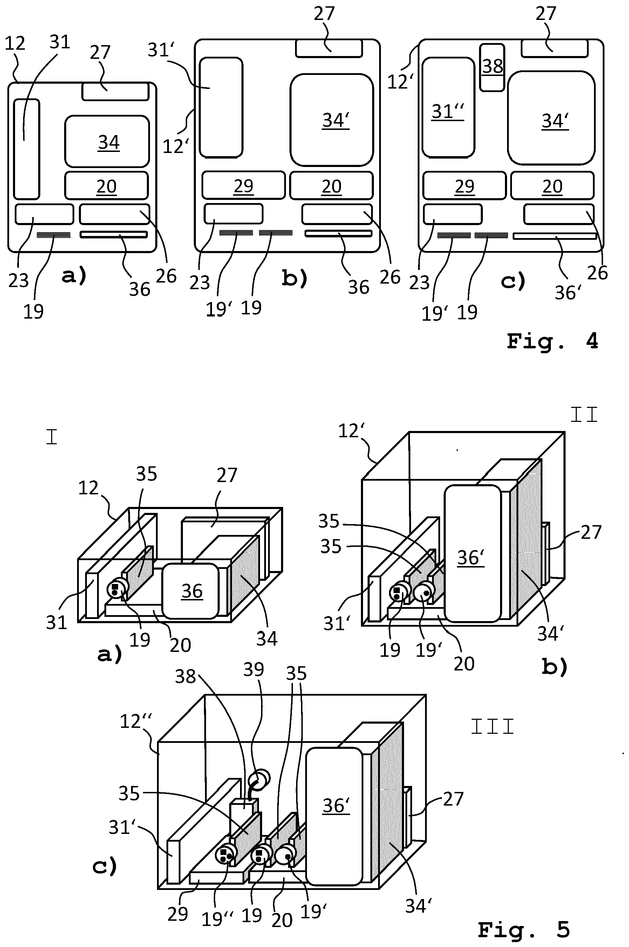

In Figur 4a-c sind verschiedene Generatortypen dargestellt mit ihren jeweiligen Funktionsblöcken. Bei dem in Figur 4a dargestellten Generatortyp handelt es sich um eine Grundausführung. Sie umfasst als Basismodule 2 ein Verteilungsmodul 20, ein Displayansteuerungsmodul 26 sowie ein Frontseitenmodul 23. Ferner sind als Individualmodule 3 vorgesehen ein Modul für die Leistungsversorgung 31, ein Modul für Inverter 34, ein Rückseitenmodul 27 sowie ein Displaymodul 36, das in der Frontplatte angeordnet ist. Ferner an der Frontplatte angeordnet ist der Ausgangsanschluss 19 für das elektrochirurgische Instrument 99 (s. Fig. 1). In Figur 4b ist ein anderer Generatortyp dargestellt, der sich von den Figur 4a dargestellten im Wesentlichen unterscheidet durch ein stärkeres Leistungsversorgungsmodul 31', ein größeres und leistungsstärkeres Modul für den Inverter 34` sowie einen zweiten Ausgangsanschluss 19`. Für die beiden Ausgangsanschlüsse 19, 19' ist, um deren jeweiligen Ausgangsmodule 35 (in Figur 4 nicht dargestellt) aufzunehmen, das Verteilungsmodul 20 ergänzt um eine Erweiterungseinheit 29, um so mindestens einen zusätzlichen Aufnahmeplatz mit einem Konnektor 22 (in Figur 4 nicht dargestellt) bereitzustellen. Um hierfür ausreichend Raum zu bieten, ist ein größeres Gehäuse 12' vorgesehen. Man erkennt, dass in der Mitte ein Freiraum verbleibt, der ggf. dank des modularen Konzepts wahlweise zur weiteren Ausrüstung mit Individualmodulen 3 oder einem Optionalmodul 14 (jeweils in Figur 4b nicht dargestellt) genutzt werden kann. In Figur 4c ist wiederum ein anderer Generatortyp dargestellt. Dieser unterscheidet sich von dem in Figur 4b dargestellten im Wesentlichen dadurch, dass ein zusätzliches Individualmodul 3 in Gestalt eines Ultraschallmoduls 38 vorgesehen ist. Um auch dieses ausreichend mit Leistung zu versorgen, ist anderes Modul für die Leistungsversorgung, nämlich eine verstärkte Leistungsversorgung 31", vorgesehen.

In Figur 5a-c sind drei verschiedene Baureihen von Generatortypen I, II und III in einer perspektivischen Ansicht dargestellt. Sie dienen zur Verdeutlichung der Anordnung der Module in verschiedenen Gehäusen. Die in Fig. 5a dargestellte erste Baureihe I ist eine Basisausführung. In einem eher kleineren Gehäuse 12 sind angeordnet ein Modul für die Leistungsversorgung 31 auf der einen Seite, ein Invertermodul 34 auf der anderen Seite als Individualmodule 3 und dazwischen das Verteilungsmodul 20 als Basismodul 2. An der Frontseite ist eine modular ausgeführte Frontplatte mit einem Displaymodul 36 angeordnet. An der Rückseite des Displaymoduls 36 ist ein Displayansteuerungsmodul 26 angeordnet (in Fig. 5a-c nicht sichtbar, vgl. Fig. 4a-c). An einer Rückwand des Gehäuses 12 ist ein Rückseitenmodul 27 zum Anschluss von externen Komponenten vorgesehen. In das Verteilungsmodul 20 eingesteckt ist ein Anschlussmodul 35, das die vom Inverter erzeugte Ausgangsenergie über einen Ausgangsanschluss 19 ausgibt.

In Fig. 5b ist eine mittlere Baureihe II dargestellt. Sie weist gegenüber der Baureihe I ein anderes Individualmodul für den Inverter aus, nämlich eines mit einem leistungsstärkeren Inverter 34`. Ferner ist ein größeres Displaymodul 36` vorgesehen, das von dem Displayansteuerungsmodul 26 angesteuert ist. All dies bedingt ein größeres Gehäuse 12`. In das Basismodul 2 ist zusätzlich ein weiteres Anschlussmodul 35 eingesteckt, das Ausgangsenergie über einen weiteren Ausgangsanschluss 19' ausgibt.

In Fig. 5c ist eine höherwertige Baureihe III dargestellt, die ähnlich zu der Baureihe II ist, aber einen weiteren Ausgangsanschluss 19" aufweist. Dazu ist ein weiteres Anschlussmodul 35 vorgesehen, welches in eine Erweiterungseinheit 29 für das Basismodul 2 eingesteckt ist. Ferner ist als Optionalmodul 14 ein Ultraschallmodul 38 vorgesehen, welches in dem Freiraum zwischen dem Modul für Leistungsversorgung 31` und dem Invertermodul 34` angeordnet ist. Es ist über Kabelverbindungen (nicht dargestellt) an das Kommunikationsnetzwerk 4 und das Stromversorgungsnetz 5 angeschlossen. All dies bedingt ein großes Gehäuse 12". An dessen Rückseite ist zur Ausgabe der Ultraschall-Energie ein zusätzlicher HF-Ausgangsanschluss 39 vorgesehen, an den das Ultraschallmodul 38 angeschlossen ist zur Ausgabe von Ultraschall-Energie.

Wie man an diesen Beispielen erkennt, können so mit wenigen Modulen 2, 3 und geringen Änderungen eine Vielzahl von unterschiedlichen Generatortypen und Baureihen erzeugt werden.

## Patentansprüche

1. Modulares Generatorsystem umfassend wenigstens zwei Generatortypen von Elektrochirurgie-Generatoren (1), die sich bezüglich ihrer Ausrüstung und/oder Funktionen unterscheiden, wobei die Generatortypen jeweils zur Speisung mindestens eines elektrochirurgischen Instruments (99) ausgebildet sind und zusammengesetzt sind aus mehreren Generatorkomponenten umfassend eine Leistungsversorgung (31) und einen Inverter (34), gespeist von der Leistungsversorgung (31) zur Erzeugung einer Ausgangsenergie, die zu mindestens einem Ausganganschluss (19) zum Anschluss des elektrochirurgischen Instruments (99) geführt ist, **dadurch gekennzeichnet, dass**
die Generatortypen jeweils in Modulbauweise ausgeführt sind mit einer Mehrzahl von Modulen, die umfassen
- mindestens ein bei den verschiedenen Generatortypen einheitliches Basismodul (2), das mindestens eine Kommunikationseinheit (40) zur Intermodulkommmunikation umfasst, und
- mindestens ein Individualmodul (3), das je nach Generatortyp unterschiedlich ist, wobei das oder die Individualmodule (3) ausgeführt sind zumindest als Modul für die Leistungsversorgung (31) und/oder Modul für den Inverter (34) und/oder mindestens ein Modul (35) für den Ausgangsanschluss (19),
wobei das mindestens eine Basismodul (2) gleich ausgebildet ist für die verschiedenen Generatortypen, und die verschiedenen Generatortypen sich hinsichtlich ihrer Individualmodul-Ausstattung unterscheiden.

2. Modulares Generatorsystem nach Anspruch 1, **dadurch gekennzeichnet, dass** als Basismodule (2) vorgesehen sind ein Displayansteuerungsmodul (26), internes Verteilungsmodul (20), und/oder Extern-Kommunikationsmodul (23) für die Kommunikation nach außen.

3. Modulares Generatorsystem nach einem der vorangehenden Ansprüche, **dadurch gekennzeichnet, dass** mehrere verschiedene Individualmodule (3) vorgesehen sind, die sich funktional unterscheiden und so Arten von Individualmodulen bilden, wobei vorzugsweise Individualmodule (3) einzeln austauschbar sind.

4. Modulares Generatorsystem nach dem vorhergehenden Anspruch, **dadurch gekennzeichnet, dass** mindestens innerhalb einer Art von Individualmodulen (3) unterschiedliche Varianten (3', 3") vorgesehen sind, insbesondere verschiedene Größen von Leistungsversorgungen und/oder Invertern.

5. Modulares Generatorsystem nach einem der vorangehenden Ansprüche, **dadurch gekennzeichnet, dass** Individualmodule (3) vorgesehen sind für Leistungsversorgung (31); Inverter (34); Anschlüsse (35), insbesondere Steckanschlüsse für elektrochirurgische Instrumente (99); und/oder Frontplatten (23); insbesondere einschließlich Anzeige und/oder Display (36).

6. Modulares Generatorsystem nach einem der vorangehenden Ansprüche, **dadurch gekennzeichnet, dass** zusätzlich mindestens ein Optionalmodul (14) mit zusätzlicher Funktionalität vorgesehen ist, das wahlfrei hinzufügbar ist, wobei vorzugsweise im montierten Zustand selbsttätig eine Stromversorgung und Kommunikationsverbindungen mit anderen Modulen (2, 3) hergestellt sind.

7. Modulares Generatorsystem nach einem der vorangehenden Ansprüche, **dadurch gekennzeichnet, dass** das modulare Generatorsystem mehrere verschiedene Baureihen (I, II, III) von Generatortypen umfasst, für die jeweils eigene Individualmodule (3) vorgesehen sind, die sich von den Individualmodulen einer anderen Baureihe unterscheiden, wobei vorzugsweise das Basismodul (2) baureihenübergreifend einsetzbar ist.

8. Modulares Generatorsystem nach einem der Ansprüche 2 bis 7, **dadurch gekennzeichnet, dass** mindestens eines der Basismodule (2) eine Stromversorgungsverteilungseinheit für andere Module (2, 3) und/oder Konnektoren (22) für das mindestens eine Modul (35) für den Ausgangsanschluss (19) aufweist, wobei an den Konnektoren (22) Anschlüsse für ein Kommunikationsnetzwerk (4), ein Stromversorgungsnetz (5) und/oder vorzugsweise auch die Ausgangsenergie-Leitung (9) aufweist.

9. Modulares Generatorsystem nach einem der vorangehenden Ansprüche, **dadurch gekennzeichnet, dass** die Module (2, 3) jeweils eine eigene Kommunikationseinheit (40) aufweisen, die im montierten Zustand mit den Kommunikationseinheiten der anderen Module (2, 3) ein Kommunikationsnetzwerk (4) zur Intermodulkommunikation bilden, das vorzugsweise selbstkonfigurierend ist,
wobei vorzugsweise zumindest die Individualmodule (3) in Bezug auf ihre Kommunikationseinheiten (40) und ihre Stromversorgung einheitliche Schnittstellen nach außen zu anderen Modulen (2, 3) aufweisen.

10. Modulares Generatorsystem nach einem der vorangehenden Ansprüche, **dadurch gekennzeichnet, dass** für das Basismodul (2) eine optionale Erweiterungseinheit (29) vorgesehen ist, die vorzugsweise wie das Basismodul (2) hochspannungsführend für die Ausgangsenergie ist.

11. Modulares Generatorsystem umfassend wenigstens zwei Generatortypen von Elektrochirurgie-Generatoren (1), die sich bezüglich ihrer Funktionen unterscheiden,
wobei die Generatortypen jeweils zur Speisung eines elektrochirurgischen Instruments (99) ausgebildet sind und zusammengesetzt sind aus mehreren Generatorkomponenten umfassend eine Leistungsversorgung (31) und einen Inverter, gespeist von der Leistungsversorgung (31) zur Erzeugung einer Ausgangsenergie, die zu mindestens einem Ausgangsanschluss (19) zum Anschluss des elektrochirurgischen Instruments (99) geführt ist,
**dadurch gekennzeichnet, dass**
das modulare Generatorsystem umfasst:
• eine Anzahl verschiedener Invertermodule (34, 34`), woraus eines für den jeweiligen Generatortyp ausgewählt und montiert ist, und
• eine Anzahl verschiedener Ausgangsanschlüsse (19), von denen mindestens einer ausgewählt und in eine Frontplatte mechanisch aufgenommen sowie mit dem montierten Invertermodul (34) elektrisch verbunden ist,
wobei jeder Elektrochirurgie-Generator (1) aufweist einen Modul-Detektor (6), der zur Erkennung montierter und angeschlossener Module (2, 3) ausgebildet ist und daraus eine real vorhandene Modulkonfiguration ermittelt.

12. Modulares Generatorsystem nach Anspruch 11, **dadurch gekennzeichnet, dass** die Module (2, 3) eine eigene Selbstkonfigurationseinheit (61) aufweisen, die dazu ausgebildet ist, das jeweilige Modul (2 ,3) abhängig von der ermittelten real vorhandenen Modulausrüstung zu konfigurieren.

13. Modulares Generatorsystem nach Anspruch 11 oder 12,
**dadurch gekennzeichnet, dass** der Modul-Detektor (6) als eine zentrale Einheit für den jeweiligen Elektrochirurgie-Generator ausgeführt ist oder dezentral auf den jeweiligen Modulen (2, 3) ausgeführt ist.

14. Modulares Generatorsystem nach einem der Ansprüche 11 bis 13, **dadurch gekennzeichnet, dass** ferner eine Freischalt-Einheit (7) vorgesehen ist, die dazu ausgebildet ist abhängig von der real vorhandenen Modulkonfiguration freigegebene Funktionen zu ermitteln und entsprechende Freischaltsignale an die Module (2, 3) zu übermitteln, wobei vorzugsweise die Freischalt-Einheit (7) als eine zentrale Einheit für den jeweiligen Elektrochirurgie-Generator (1) oder dezentral auf den jeweiligen Modulen (2, 3) ausgeführt ist.

15. Modulares Generatorsystem nach einem der vorangehenden Ansprüche, **dadurch gekennzeichnet, dass** die Module (2 ,3) dazu ausgebildet sind, dass im montierten Zustand selbsttätig Kommunikationsverbindungen und eine Stromversorgung der Module (2, 3) hergestellt sind, wobei vorzugsweise die Kommunikationsverbindungen ein Kommunikationsnetzwerk (4) sind, wobei weiter vorzugsweise zusätzliche Signalverbindungen vorgesehen sind für Freigabesignale bezüglich Aktivität der Module (2, 3).

16. Modulares Generatorsystem nach einem der vorangehenden Ansprüche, **dadurch gekennzeichnet, dass** die Module (2, 3) jeweils aufweisen eine eigene Prozessoreinheit (80) mit einem eigenen Steuerprogramm als eigenständige lokale Betriebssteuerung für das jeweilige Modul (2, 3), und sie angeschlossen sind an ein generatorinternes Stromversorgungsnetz (5) sowie Kommunikationsnetzwerk (4), wobei vorzugsweise zumindest die Invertermodule (34) und/oder optionale Ultraschallmodule (38) einen zusätzlichen Hochfrequenz-Ausgangsanschluss aufweisen (39).

17. Modulares Generatorsystem nach einem der vorangehenden Ansprüche, **dadurch gekennzeichnet, dass** ferner eine Anzahl verschiedener Leistungsversorgungsmodule (31, 31', 31") vorgesehen sind, woraus eines für den jeweiligen Generatortyp ausgewählt und montiert ist, und/oder eine Sicherungs-Einheit (82) vorgesehen ist, die dazu ausgebildet ist eine real vorhandene Modulkonfiguration mit einem eindeutigen, fest eingespeicherten Sollausrüstungsmerkmal (83) für die Modulkonfiguration zu vergleichen und bei Abweichung ein Fehlersignal auszugeben, das vorzugsweise eine Betriebssperre betätigt, und/oder eine Upgrade-Einheit (84) vorgesehen ist, die von extern über eine gesicherte Kommunikationsverbindung (85) ansteuerbar ist und mit der Sicherungs-Einheit (82) und der Selbstkonfigurationseinheit (61) der Module (2,3) zusammenwirkt, um das eingespeicherte Sollausrüstungskennungsmerkmal (83) mit einer autorisierten Änderung zu versehen und/oder zusätzliche Funktionen freizuschalten.
